(19) Europäisches Patentamt
European Patent Office
Office européen des brevets

(11) EP 2 156 833 B1

(12) **EUROPEAN PATENT SPECIFICATION**

(45) Date of publication and mention of the grant of the patent:
**30.11.2011 Bulletin 2011/48**

(21) Application number: **08752538.2**

(22) Date of filing: **09.05.2008**

(51) Int Cl.:
***A61K 31/428*** *(2006.01)* ***A61P 25/02*** *(2006.01)*
***A61P 27/02*** *(2006.01)* ***A61P 43/00*** *(2006.01)*
***A61K 45/00*** *(2006.01)* ***C07D 277/82*** *(2006.01)*

(86) International application number:
**PCT/JP2008/058655**

(87) International publication number:
**WO 2008/140051 (20.11.2008 Gazette 2008/47)**

(54) **PROPHYLACTIC OR THERAPEUTIC AGENT FOR POSTERIOR OCULAR DISEASE COMPRISING NON-ERGOT SELECTIVE D2 RECEPTOR AGONIST AS ACTIVE INGREDIENT**

PROPHYLAKTISCHES ODER THERAPEUTISCHES MITTEL FÜR POSTERIORE AUGENERKRANKUNG MIT SELEKTIVEM NICHT-ERGOT-D2-REZEPTORAGONIST ALS WIRKSTOFF

AGENT PROPHYLACTIQUE OU THÉRAPEUTIQUE DESTINÉ À UNE MALADIE OCULAIRE POSTÉRIEURE COMPRENANT UN AGONISTE QUI N'EST PAS DÉRIVÉ DE L'ERGOT DE SEIGLE SÉLECTIF DU RÉCEPTEUR D2 EN TANT QUE PRINCIPE ACTIF

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **11.05.2007 JP 2007126232**

(43) Date of publication of application:
**24.02.2010 Bulletin 2010/08**

(73) Proprietor: **Santen Pharmaceutical Co., Ltd**
**Osaka-shi**
**Osaka 533-8651 (JP)**

(72) Inventors:
- **OKAMOTO, Kazuyoshi**
**Ikoma-shi**
**Nara 630-0101 (JP)**
- **SHIBAGAKI, Keiichi**
**Ikoma-shi**
**Nara 630-0101 (JP)**

(74) Representative: **Müller-Boré & Partner Patentanwälte**
**Grafinger Straße 2**
**81671 München (DE)**

(56) References cited:
**WO-A1-91/01136 WO-A1-2007/022182**
**WO-A2-2007/121188 JP-A- 2004 323 486**
**JP-A- 2006 176 499**

- **DANZEISEN R. ET AL.: 'Targeted antioxidative and neuroprotective properties of the dopamine agonist pramipexole and its nondopaminergic enantiomer SND919CL2x [(+)2-amino-4,5,6,7-tetrahydro-6-L-propylam ino-benzathiazole dihydrochloride]' JOURNAL OF PHARMACOLOGY AND EXPERIMENTAL THERAPEUTICS vol. 316, no. 1, 2006, pages 189 - 199, XP008125303**
- **KONO Y. ETAL.: 'Parkinson's Disease Chiryoyaku Ensan Pramipexole (BI.Sifrol Tablets) no Yakuri Sayo to Rinsho Seiseki' FOLIA PHARMACOLOGICA JAPONICA vol. 123, no. 6, 01 June 2004, pages 429 - 440, XP008125302**
- **MIZUNO Y. ET AL.: 'Parkinson's Disease ni Taisuru SND 919 (pramipexole) no Choki Toyo Shiken' NEUROLOGICAL THERAPEUTICS vol. 20, no. 4, 25 July 2003, pages 465 - 477, XP008141300**
- **NIPPON BOEHRINGER INGELHEIM CO., LTD.: 'Tenpu Bunsho (BI.Sifrol Tablets o.125mg BI.Sifrol Tablets 0.5mg)' November 2006, pages 1 - 4, XP008118265**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 156 833 B1

Remarks:

The file contains technical information submitted after the application was filed and not included in this specification

## Description

Technical Field

The present invention relates to a prophylactic or therapeutic agent for a posterior ocular disease containing a selective $D_2$ receptor agonist as an active ingredient, wherein the agonist is a non-ergot agonist.

Background Art

Dopamine receptors specifically bind to dopamine and function to allow dopamine to exhibit its action and are distributed in brain dopaminergic neurons. Dopamine receptors are mainly divided into two receptor subfamilies based on their association with adenylate cyclase. That is, dopamine receptors are divided into the $D_1$ receptor subfamily ($D_1$ and $D_5$ receptors) in which a receptor is associated with adenylate cyclase in a stimulatory manner via a stimulatory G-protein (Gs) and the $D_2$ receptor subfamily ($D_2$, $D_3$ and $D_4$ receptors) in which a receptor acts on adenylate cyclase in an inhibitory manner via an inhibitory G-protein (Gi) and is associated with metabolism of phosphatidylinositol and release of arachidonic acid in a stimulatory manner or is associated with potassium channel or calcium channel.

The selective $D_2$ receptor agonist is a compound which selectively binds to a $D_2$ receptor subfamily member and functions to allow the member to exhibit its action. The agonist has been used for treatment of Parkinson's disease as well as levodopa (L-dopa) which is a dopamine precursor and has been reported to be more useful than levodopa in several aspects. For example, it has been reported that: the agonist does not need to be enzymatically converted for exhibiting drug activity and therefore is not dependent on the functional capacity of the nigrostriatal neurons; and the agonist often has duration of action longer than levodopa and therefore is often useful in the management of dose-related fluctuation in a motor state. Further, the selective $D_2$ receptor agonist has selective activity unlike levodopa which has activity against all types of dopamine receptors in the entire brain, and the agonist has relative selectivity for different dopamine receptor subtypes. For example, pramipexole or the like has selective activity against $D_2$ receptor subfamily members and hardly acts on $D_1$ receptor subfamily members. Incidentally, pramipexole has been launched as a therapeutic agent for Parkinson's disease.

On the other hand, there are Non-patent documents 1 to 3 as reports suggesting that dopamine participates in neovascularization or vascular hyperpermeability. In Non-patent document 1, the effect of dopamine on vascular hyperpermeability or neovascularization was studied using animal models (mice) to which MOT (mouse ovarian tumor) was intraperitoneally administered. This document reports that dopamine suppressed vascular hyperpermeability and neovascularization via VEGF, and also promoted VEGFR-2 endocytosis via a $D_2$ receptor and suppressed receptor binding of VEGF and phosphorylation of VEGFR-2. Non-patent document 3 reports that dopamine and tyrosine hydroxylase were not found in human and rat stomach cancer tissues, and dopamine suppressed tumor neovascularization by inhibiting phosphorylation of VEGFR-2 in tumor vascular endothelial cells. Non-patent document 2 reports that, in peripheral dopaminergic nerve-ablated mice, an increase in neovascularization via VEGF and the growth of malignant tumors due to an increase in microvascular permeability were observed.

Further, pramipexole is a selective $D_2$ receptor agonist and has been launched as a therapeutic agent for Parkinson's disease as described above. There are Patent documents 1 and 2 as reports regarding pramipexole other than the above-mentioned documents. Patent document 2 describes pramipexole and that the compound is useful for Parkinson's disease and central nervous system neuropsychiatric diseases. Patent document 1 suggests the use of pramipexole for treating glaucoma.

However, there is no report on study of an inhibitory effect on neovascularization, a suppressive effect on photoreceptor cell damage or a suppressive effect on vascular hyperpermeability in a posterior ocular tissue such as choroid or retina as a report regarding a non-ergot selective $D_2$ receptor agonist typified by pramipexole. Further, there is no report on study of the pharmacological effect of the agonist on a posterior ocular disease, and particularly there is no report on study of the prophylactic or improvement effect thereof on age-related macular degeneration, diabetic retinopathy, diabetic macular edema or the like.

Age-related macular degeneration is a disease characterized in that retinal tissues in the macular region are impaired with aging, leading to visual impairment, and is one of the causes of visual impairment in the elderly which may result in visual loss. Age-related macular degeneration can be classified into three major disease types: (1) early age-related macular degeneration characterized by drusen formation and pigmentation or depigmentation (sometimes considered to be a precursor lesion of age-related macular degeneration); (2) atrophic age-related macular degeneration, the nature of which is atrophic degeneration of retinal pigment epithelial cells in the macular region and atrophic degeneration of retinal photoreceptor cells secondary to atrophic degeneration of retinal pigment epithelial cells; and (3) exudative age-related macular degeneration in which new blood vessels from the choroid grow beneath the retina in the macular region and hemorrhage or cell exudation occurs.

Patent document 1: WO 91/01136
Patent document 2: JP-B-05-72907
Non-patent document 1: Nature Med., 7, 569-574 (2001)
Non-patent document 2: Cancer Res., 64, 5551-5555 (2004)
Non-patent document 3: Clinical Cancer Research, 10, 4349-4356 (2004)

Disclosure of the Invention

Problems to be Solved by the Invention

Accordingly, it is an interesting object to search a new medicinal use of a non-ergot agonist among selective $D_2$ receptor agonists.

A method for treating atrophic age-related macular degeneration has been studied in various ways, however, a clinically effective treatment method has not been established yet. Further, a method for treating exudative age-related macular degeneration has also been studied in various ways, however, an effective treatment method which enables complete restoration of decreased visual acuity has not been found yet. Therefore, to find a therapeutic agent for age-related macular degeneration which is a low molecular weight compound and can be orally administered at low cost, particularly a therapeutic agent effective for atrophic age-related macular degeneration is of extremely great significance clinically.

Further, a method for treating diabetic retinopathy or diabetic macular edema has been vigorously studied, however, a therapeutic agent with high clinical efficacy has not been found yet. Therefore, to find a therapeutic agent for these diseases is of great significance.

Means for Solving the Problems

The present inventors conducted intensive studies to search a new medicinal use of a selective $D_2$ receptor agonist which is non-ergot agonist and found that pramipexole which is the agonist has excellent inhibitory effect on neovascularization, suppressive effect on photoreceptor cell damage and suppressive effect on vascular hyperpermeability in a posterior ocular tissue such as choroid or retina, and thus the invention was achieved. On the other hand, bromocriptine which is a selective $D_2$ receptor agonist and is ergot agonist, and dopamine which is a non-selective dopamine receptor agonist were evaluated for an inhibitory effect on neovascularization. However, these compounds were found to have little or no inhibitory effect on neovascularization in the choroid or retina. Further, bromocriptine was evaluated for a suppressive effect on photoreceptor cell damage in the same manner and found to have no suppressive effect on photoreceptor cell damage. That is, these results show that a non-ergot selective $D_2$ receptor agonist typified by pramipexole exhibits a far higher pharmacological effect than an ergot selective $D_2$ receptor agonist and a non-selective dopamine receptor agonist and support the excellent usefulness of the non-ergot $D_2$ receptor agonist.

Further, the three compounds of pramipexole, bromocriptine and dopamine have different affinity for dopamine receptor subtypes, however, they are all dopamine receptor agonists and pharmacologically have the same activity. However, as described above, it is only pramipexole that the inhibitory effect on neovascularization in the choroid or retina was observed, and in the case of bromocriptine and dopamine, the inhibitory effect was hardly or not at all observed. Further, when the suppressive effects on photoreceptor cell damage of pramipexole and bromocriptine were compared, it was found that only pramipexole has a marked suppressive effect on photoreceptor cell damage and bromocriptine has little or no suppressive effect on photoreceptor cell damage. In particular, it is surprising that, although pramipexole and bromocriptine are both selective $D_2$ receptor agonists, there is a clear difference in the pharmacological effects between these two compounds with respect to the inhibitory effect on neovascularization in the choroid or retina and the suppressive effect on photoreceptor cell damage.

That is, the invention relates to a prophylactic or therapeutic agent for a posterior ocular disease such as age-related macular degeneration, diabetic retinopathy, diabetic macular edema, retinitis pigmentosa, proliferative vitreoretinopathy, retinal artery occlusion, retinal vein occlusion, uveitis, Leber's disease, retinopathy of prematurity, retinal detachment, retinal pigment epithelial detachment, central serous chorioretinopathy, central exudative chorioretinopathy, polypoidal choroidal vasculopathy, multifocal choroiditis, neovascular maculopathy, retinal artery macroaneurysm, optic nerve damage caused by any of these diseases, optic nerve damage caused by glaucoma or ischemic optic nerve damage, containing, as an active ingredient, a selective $D_2$ receptor agonist wherein the agonist is a non-ergot agonist (hereinafter also collectively referred to as "present compound").

The "salt" of the present compound is not particularly limited as long as it is a pharmaceutically acceptable salt, and examples thereof include salts with an inorganic acid such as hydrochloric acid, hydrobromic acid, hydroiodic acid, nitric acid, sulfuric acid or phosphoric acid; and salts with an organic acid such as acetic acid, fumaric acid, maleic acid, succinic acid, citric acid, tartaric acid, adipic acid, gluconic acid, glucoheptonic acid, glucuronic acid, terephthalic acid,

methanesulfonic acid, lactic acid, hippuric acid, 1,2-ethanedisulfonic acid, isethionic acid, lactobionic acid, oleic acid, pamoic acid, polygalacturonic acid, stearic acid, tannic acid, trifluoromethanesulfonic acid, benzenesulfonic acid, p-toluenesulfonic acid, lauryl sulfate, methyl sulfate, naphthalene sulfonate or sulfosalicylic acid.

Further, the present compound may be in the form of a hydrate or a solvate.

The "selective $D_2$ receptor agonist" as used herein refers to an agonist or stimulant which binds to a $D_2$ receptor subfamily member (a $D_2$, $D_3$ or $D_4$ receptor) and has a higher affinity for a $D_2$ receptor subfamily member than that for a $D_1$ receptor subfamily member (a $D_1$ or $D_5$ receptor). Further, the "ergot agonist" refers to an agonist which has an ergot alkaloid backbone in the chemical structure, and the "non-ergot agonist" refers to an agonist which does not have an ergot alkaloid backbone in the chemical structure. Incidentally, the ergot alkaloid refers to an indole alkaloid contained in the sclerotium of Claviceps purpurea that mainly parasitizes rye.

The present compound include pramipexole hydrochloride represented by the following chemical structural formula.

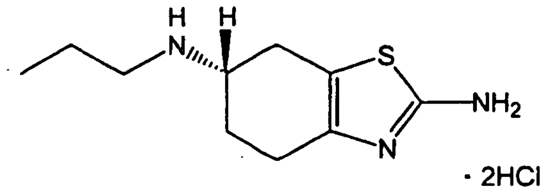

The posterior ocular disease as used herein refers to a disease in the vitreous body, retina, choroid, sclera or optic nerve, and examples thereof include eye diseases such as age-related macular degeneration (drusen formation in early age-related macular degeneration, atrophic age-related macular degeneration, exudative age-related macular degeneration), diabetic retinopathy (simple diabetic retinopathy, preproliferative diabetic retinopathy, proliferative diabetic retinopathy), diabetic macular edema, retinitis pigmentosa, proliferative vitreoretinopathy, retinal artery occlusion, retinal vein occlusion, uveitis, Leber's disease, retinopathy of prematurity, retinal detachment, retinal pigment epithelial detachment, central serous chorioretinopathy, central exudative chorioretinopathy, polypoidal choroidal vasculopathy, multifocal choroiditis, neovascular maculopathy, retinal artery macroaneurysm, optic nerve damage caused by any of these diseases, optic nerve damage caused by glaucoma and ischemic optic nerve damage. Preferred examples thereof include eye diseases such as age-related macular degeneration (particularly exudative age-related macular degeneration and/or atrophic age-related macular degeneration), diabetic retinopathy and diabetic macular edema. Incidentally, it is obvious from the known documents (Journal of Japanese Ophthalmological Society, 103, 923-947 (1999), and Shin Zusetsu Rinsho Ganka Koza (New Illustrated Handbook of Clinical Ophthalmology), Vol. 5, "Vitreoretinal Diseases", First edition, edited by Y. Tano, MEDICAL VIEW, pp. 184-189 and 232-237, (2000)) that inhibition of neovascularization or suppression of vascular hyperpermeability in a posterior ocular tissue such as choroid or retina is useful for treatment of the above-mentioned diseases. Further, it is obvious from the known document (Ohan Shikkan Tekisuto & Atorasu (Macular Disease Textbook and Atlas), First Edition, edited by M. Uyama, et al., Igaku-Shoin Ltd., pp. 43-45) that suppression of photoreceptor cell damage is useful for age-related macular degeneration among the above-mentioned diseases.

The present compound can be formulated into a single preparation or a combination preparation by adding a pharmaceutically acceptable additive as needed using a widely used technique.

When the present compound is used for prophylaxis or therapy of the above-mentioned eye diseases, it can be administered to a patient orally or parenterally. Examples of the route of administration include oral administration, topical administration to eyes (such as instillation administration, administration into conjunctival sac, intravitreal administration, subconjunctival administration and sub-Tenon's administration), intravenous administration and transdermal administration. Further, the present compound is formulated into a dosage form suitable for administration along with a pharmaceutically acceptable additive as needed. Examples of the dosage form suitable for oral administration include tablets, capsules, granules and powders, and examples of the dosage form suitable for parenteral administration include injections, eye drops, ophthalmic ointments, patches, gels and inserts. These can be prepared using a common technique widely used in this field. Further, the present compound can also be formulated into a preparation for intraocular implant or a DDS (drug delivery system) preparation such as a microsphere other than these preparations.

For example, a tablet can be prepared by properly selecting and using an excipient such as lactose, glucose, D-mannitol, anhydrous calcium hydrogen phosphate, starch or sucrose; a disintegrant such as carboxymethyl cellulose, carboxymethyl cellulose calcium, croscarmellose sodium, crosspovidone, starch, partially pregelatinized starch or low-substituted hydroxypropyl cellulose; a binder such as hydroxypropyl cellulose, ethyl cellulose, gum arabic, starch, partially pregelatinized starch, polyvinyl pyrrolidone or polyvinyl alcohol; a lubricant such as magnesium stearate, calcium stearate, talc, hydrous silicon dioxide or a hydrogenated oil; a coating agent such as purified sucrose, hydroxypropylmethyl

cellulose, hydroxypropyl cellulose, methyl cellulose, ethyl cellulose or polyvinyl pyrrolidone; a corrigent such as citric acid, aspartame, ascorbic acid or menthol; or the like.

An injection can be prepared by selecting and using a tonicity agent such as sodium chloride; a buffer such as sodium phosphate; a surfactant such as polyoxyethylene sorbitan monooleate; a thickening agent such as methyl cellulose; or the like as needed.

An eye drop can be prepared by selecting and using a tonicity agent such as sodium chloride or concentrated glycerin; a buffer such as sodium phosphate or sodium acetate; a surfactant such as polyoxyethylene sorbitan monooleate, polyoxyl 40 stearate or polyoxyethylene hydrogenated castor oil; a stabilizer such as sodium citrate or sodium edetate; a preservative such as benzalkonium chloride or paraben; or the like as needed. The pH of the eye drop is permitted as long as it falls within the range that is acceptable as an ophthalmic preparation, but is generally preferably in the range of from 4 to 8. Further, an ophthalmic ointment can be prepared with a widely used base such as white petrolatum or liquid paraffin.

An insert can be prepared by pulverizing and mixing a biodegradable polymer such as hydroxypropyl cellulose, hydroxypropylmethyl cellulose, a carboxy vinyl polymer or polyacrylic acid along with the present compound and compression molding the resulting powder. If necessary, an excipient, a binder, a stabilizer or a pH adjusting agent can be used. A preparation for intraocular implant can be prepared using a biodegradable polymer such as poiylactic acid, polyglycolic acid, a lactic acid-glycolic acid copolymer or hydroxypropyl cellulose.

The present invention also relates to a method for prophylaxis or therapy of a posterior ocular disease, comprising administering a pharmaceutically effective amount of pramipexole or a salt thereof to a patient.

The dose of the present compound can be properly changed depending on the dosage form, severity of symptoms, age or body weight of a patient to which the present compound is to be administered, medical opinion and the like. In the case of oral administration, the present compound can be generally administered to an adult once or divided into several times at a dose of from 0.01 to 5000 mg, preferably from 0.1 to 2500 mg, more preferably from 0.5 to 1000 mg per day. In the case of an injection, the present compound can be generally administered to an adult once or divided into several times at a dose of from 0.0001 to 2000 mg per day. In the case of an eye drop or an insert, generally a preparation containing the active ingredient in an amount of from 0.000001 to 10% (w/v), preferably from 0.00001 to 1% (w/v), more preferably from 0. 0001 to 0.1% (w/v) can be administered once or several times per day. Further, in the case of a patch, a patch containing the active ingredient in an amount of from 0.0001 to 2000 mg can be applied to an adult, and in the case of a preparation for intraocular implant, a preparation for intraocular implant containing the active ingredient in an amount of from 0.0001 to 2000 mg can be implanted in the eye of an adult.

Advantageous effects of the Invention

When a test described below was performed, it was shown, in a pharmacological test, that pramipexole hydrochloride which is the present compound (hereinafter also referred to as "Compound A") significantly inhibits choroidal neovascularization in rat models of laser-induced choroidal neovascularization. That is, a non-ergot selective $D_2$ receptor agonist typified by Compound A is useful as a prophylactic or therapeutic agent for a posterior ocular disease accompanied by neovascularization such as age-related macular degeneration, particularly exudative age-related macular degeneration or the like.

On the other hand, bromocriptine mesilate which is an ergot selective $D_2$ receptor agonist (hereinafter also referred to as "Compound B") and dopamine hydrochloride which is a non-selective dopamine receptor agonist (hereinafter also referred to as "Compound C") were also subjected to the same test. However, these compounds were found to have little or no inhibitory effect on neovascularization in the choroid. That is, these results show that a non-ergot selective $D_2$ receptor agonist typified by Compound A exhibits a far higher inhibitory effect on neovascularization than an ergot selective $D_2$ receptor agonist and a non-selective dopamine receptor agonist and support the excellent usefulness of the non-ergot $D_2$ receptor agonist.

Further, in a pharmacological test described below, it was shown that Compound A significantly suppresses photoreceptor cell damage in mouse models of light damage. That is, a non-ergot selective $D_2$ receptor agonist typified by Compound A is useful as a prophylactic or therapeutic agent for a posterior ocular disease accompanied by photoreceptor cell damage such as age-related macular degeneration, particularly atrophic age-related macular degeneration or the like.

On the other hand, Compound B which is an ergot selective $D_2$ receptor agonist was also subjected to the same test. However, this compound was found to have little or no suppressive effect on photoreceptor cell damage. That is, these results show that a non-ergot selective $D_2$ receptor agonist typified by Compound A exhibits a far more potent suppressive effect on photoreceptor cell damage than an ergot selective $D_2$ receptor agonist and support the excellent usefulness of the non-ergot $D_2$ receptor agonist.

Further, in a pharmacological test described below, it was shown that Compound A significantly suppresses retinal vascular hyperpermeability in rat models of thrombin-induced retinal vascular hyperpermeability. That is, it was

shown that a non-ergot selective $D_2$ receptor agonist typified by Compound A has a marked prophylactic or improvement effect on a posterior ocular disease associated with retinal angiopathy such as diabetic retinopathy or diabetic macular edema.

Best Mode for Carrying Out the Invention

Hereinafter, results of pharmacological tests and preparation examples will be shown, however, these examples are for understanding the invention well, and are not meant to limit the scope of the present invention,

[Pharmacological Test 1]

The present compound, an ergot selective $D_2$ receptor agonist and a non-selective dopamine receptor agonist were evaluated for usefulness using rat models of laser-induced choroidal neovascularization.

(Method of creating a Rat Model of Krypton Laser-Induced Choroidal Neovascularization)

A rat was given general anesthesia by intramuscular administration of 1 ml/kg of a mixed solution of a 5% (w/v) ketamine hydrochloride injectable solution and a 2% xylazine hydrochloride injectable solution (7:1), and a 0.5% (w/v) tropicamide-0.5% phenylephrine hydrochloride ophthalmic solution was instilled into the eyes to cause mydriasis, and then, photocoagulation was performed with a krypton laser photocoagulation apparatus. The photocoagulation was performed in a posterior fundus at eight spots per eye sparsely by focusing on the retinal deep layer avoiding thick retinal blood vessels (coagulation conditions: spot size: 100 μm, output: 100 mW, coagulation time: 0.1 sec). After the photocoagulation, the ocular fundus was photographed, and the site where the laser was irradiated was confirmed.

(Drug Administration Method)

Compound A was dissolved in a 1% (w/v) methyl cellulose solution (prepared by dissolving methyl cellulose in purified water) at a concentration of 0.06 mg/ml or 0.2 mg/ml, whereby solutions of Compound A at such concentrations were prepared. Each of the solutions of Compound A at such concentrations was orally administered once daily at a dose of 0.3 mg/kg or 1 mg/kg starting from the day of photocoagulation surgery for 7 days including the day of surgery. Compound B was also orally administered in the same manner. That is, Compound B was dissolved in a 1% (w/v) methyl cellulose solution (prepared by dissolving methyl cellulose in purified water) at a concentration of 0.06 mg/ml or 0.2 mg/ml, whereby solutions of Compound B at such concentrations were prepared. Each of the solutions of Compound B at such concentrations was orally administered once daily at a dose of 0.3 mg/kg or 1 mg/kg starting from the day of photocoagulation surgery for 7 days including the day of surgery. On the other hand, Compound C was dissolved in PBS (phosphate buffer) at a concentration of 100 mg/ml, and the resulting solution of Compound C was intraperitoneally administered once daily at a dose of 100 mg/kg starting from the day of photocoagulation surgery for 7 days including the day of surgery. In a vehicle administration group, a 1% (w/v) methyl cellulose solution or PBS was administered in the same manner.

(Evaluation Method)

On day 7 after photocoagulation, each rat was given general anesthesia by intramuscular administration of 1 ml/kg of a mixed solution of a 5% (w/v) ketamine hydrochloride injectable solution and a 2% xylazine hydrochloride injectable solution (7:1), and a 0.5% (w/v) tropicamide-0.5% phenylephrine hydrochloride ophthalmic solution was instilled into the eyes to cause mydriasis, and then, 0.1 ml of a 10% fluorescein solution was injected from the tail vein, and fluorescence fundus photography was performed. In the fluorescence fundus photography, a spot where fluorescence leakage was not observed (absence of neovascularization) was judged as negative, and a spot where fluorescence leakage was observed was judged as positive (presence of neovascularization). When there are two photocoagulation sites where a little fluorescence leakage was observed, they were judged as positive (presence of neovascularization). Then, the choroidal neovascularization incidence rate (%) was calculated from the number of positive spots relative to the eight laser irradiation spots in accordance with Equation 1, and the inhibition rate (%) of the drug to be evaluated was calculated in accordance with Equation 2. The results of Compounds A to C are shown in Table 1. The case number in each administration group is 7 to 8.

[Equation 1]

Choroidal neovascularization incidence rate (%) = (Number of positive spots / total number of photocoagulation sites) x 100

[Equation 2]

$$\text{Inhibition rate (\%)} = (A_0 - A_X) / A_0 \times 100$$

$A_0$: Choroidal neovascularization incidence rate of vehicle administration group
$A_x$: Choroidal neovascularization incidence rate of drug administration group

Table 1

| Group | | Suppression rate (%) |
|---|---|---|
| Compound A | 0.3 mg/kg | 60.9 |
| | 1 mg/kg | 60.9 |
| Compound B | 0.3 mg/kg | -2.1 |
| | 1 mg/kg | 4.1 |
| Compound C | 100 mg/kg | 11.8 |

(Discussion)

As is apparent from Table 1, it was shown that Compound A inhibits choroidal neovascularization in the rat models of laser-induced choroidal neovascularization. On the other hand, in the case of Compound B which is an ergot selective $D_2$ receptor agonist and Compound C which is a non-selective dopamine receptor agonist, an inhibitory effect on neovascularization in the choroid was hardly or not at all observed. It is surprising that, although all Compounds A to C have an effect of activating a $D_2$ receptor, only Compound A which is a non-ergot selective $D_2$ receptor agonist exhibits a high inhibitory effect on neovascularization in the choroid.
From the above results, it was shown that the present compound typified by Compound A has an excellent inhibitory effect on neovascularization in the choroid and has a marked prophylactic or improvement effect on a posterior ocular disease associated with neovascularization such as age-related macular degeneration, particularly exudative age-related macular degeneration or the like.

[Pharmacological Test 2]

The present compound and an ergot selective $D_2$ receptor agonist were evaluated for usefulness using mouse models of light damage. Incidentally, a mouse model of light damage is a model animal in which damage has been induced by light exposure mainly in the photoreceptor cells and retinal pigment epithelial cell layer and is widely used mainly as a model animal of retinal degeneration (for example, age-related macular degeneration, particularly atrophic age-related macular degeneration or retinitis pigmentosa) (Invest. Ophthalmol. Vis. Sci., 2005; 46: 979-987).

(Method of creating a Mouse Model of Light Damage)

After a 0.5% (w/v) tropicamide-0.5% phenylephrine hydrochloride ophthalmic solution was instilled into the eyes of a mouse to cause mydriasis, light damage was induced in the mouse by light exposure (exposure conditions: illuminance level: 5000 Lux, exposure time: 2 hours) with an apparatus for inducing light damage (Sakami Medical Instrument).

(Drug Administration Method)

Compound A was dissolved in a 1% (w/v) methyl cellulose solution (prepared by dissolving methyl cellulose in purified water) at a concentration of 0.006, 0.02, 0.06 or 0.2 mg/ml, whereby solutions of Compound A were prepared. Each of the solutions of Compound A was orally administered once at a dose of 0.03, 0.1, 0.3 or 1 mg/kg one hour before light exposure. Compound B was also orally administered in the same manner. That is, Compound B was dissolved in a 1% (w/v) methyl cellulose solution at a concentration of 0.02 or 0.2 mg/ml, whereby solutions of Compound B were prepared. Each of the solutions of Compound B was orally administered once at a dose of 0.1 or 1 mg/kg one hour before light exposure. In a vehicle administration group, a 1% (w/v) methyl cellulose solution was administered in the same manner.

(Evaluation Method)

One day after light exposure, each mouse was given general anesthesia by intramuscular administration of 2 ml/kg of a mixed solution of a 5% (w/v) ketamine hydrochloride injectable solution, a 2% xylazine hydrochloride injectable solution and physiological saline (7:1:8), and a 0.5% (w/v) tropicamide-0.5% phenylephrine hydrochloride ophthalmic solution was instilled into the eyes to cause mydriasis. Then, the electroretinogram (ERG) was measured using portable ERG & VEP LE-3000 (Tomey Corporation) (measurement conditions: stimulus luminance: 3000 cd/$m^2$, stimulus duration: 10 msec, background light luminance: 0 cd/$m^2$), and a- and b-wave amplitudes were calculated from the obtained waveforms. Then, the suppression rate (%) of the drug to be evaluated against the decrease in a- and b-wave amplitudes (photoreceptor cell damage) caused by light exposure was calculated in accordance with Equations 3 and 4. The results of Compound A and Compound B are shown in Table 2. The case number in each administration group was 6 to 8 per group, and the mean value was used for calculating the suppression rate.

[Equation 3]

$$\text{Suppression rate against photoreceptor cell damage (\%) based on a-wave} = (A_{AZ} - A_{AY}) / (A_{AX} - A_{AY}) \times 100$$

$A_{AX}$: a-wave amplitude in normal (untreated) group
$A_{AY}$: a-wave amplitude in group with light exposure and vehicle administration
As: a-wave amplitude in group with light exposure and drug administration

[Equation 4]

$$\text{Suppression rate against photoreceptor cell damage (\%) based on b-wave} = (A_{BZ} - A_{BY}) / (A_{BX} - A_{BY}) \times 100$$

$A_{BX}$: b-wave amplitude in normal (untreated) group
$A_{BY}$: b-wave amplitude in group with light exposure and vehicle administration
$A_{BZ}$: b-wave amplitude in group with light exposure and drug administration

Table 2

| Group | | Suppression rate (%) based on a-wave | Suppression rate (%) based on b-wave |
|---|---|---|---|
| Compound A | 0.03 mg/kg | 41.4 | 49.2 |
| | 0.1 mglkg | 47.3 | 44.1 |
| | 0.3 mglkg | 70.0 | 72.1 |
| | 1 mg/kg | 102.0 | 92.1 |
| Compound B | 0.1 mglkg | 0.2 | -3.8 |

(continued)

| Group | Suppression rate (%) based on a-wave | Suppression rate (%) based on b-wave |
|---|---|---|
| 1 mg/kg | 4.8 | 4.9 |

(Discussion)

As is apparent from Table 2, it was shown that Compound A significantly suppresses photoreceptor cell damage in the mouse models of light damage. On the other hand, in the case of Compound B which is an ergot selective $D_2$ receptor agonist, a suppressive effect on photoreceptor cell damage was hardly or not at all observed. It is surprising that, although both Compounds A and B have an effect of activating a $D_2$ receptor, only Compound A which is a non-ergot selective $D_2$ receptor agonist exhibits a high suppressive effect on photoreceptor cell damage.

From the above results, it was shown that a non-ergot selective $D_2$ receptor agonist typified by Compound A has an excellent protective effect on photoreceptor cell damage and has a marked prophylactic or improvement effect on a posterior ocular disease associated with photoreceptor cell damage such as age-related macular degeneration, particularly atrophic age-related macular degeneration, retinitis pigmentosa or the like.

[Pharmacological Test 3]

The present compound was evaluated for usefulness using rat models of thrombin-induced retinal vascular hyperpermeability. Incidentally, thrombin has been reported to induce thrombus formation in the retinal blood vessels through intravitreal injection thereof (Journal of Japanese Ophthalmological Society, 1989; 93, 978-985), and the rat model is widely used as a model of a disease state accompanied by retinal angiopathy (vascular occlusion) (such as diabetic retinopathy, diabetic macular edema, retinal vein occlusion or retinal artery occlusion).

(Method of creating a Rat Model of Thrombin-Induced Retinal Vascular Hyperpermeability)

A rat was given general anesthesia by intramuscular administration of 1 ml/kg of a mixed solution of a 5% (w/v) ketamine hydrochloride injectable solution and a 2% xylazine hydrochloride injectable solution (7:1), and a 0.5% (w/v) tropicamide-0.5% phenylephrine hydrochloride ophthalmic solution was instilled into the eyes to cause mydriasis. Then, 5 μL of thrombin (600 U/mL) was injected into the vitreous body using a 33-gauge needle so as not to hurt the lens and retina. To a rat in a normal group, PBS (phosphate buffer) was injected instead of thrombin.

(Drug Administration Method)

Compound A was dissolved in a 1% (w/v) methyl cellulose solution (prepared by dissolving methyl cellulose in purified water) at a concentration of 0.2 mg/ml, whereby a solution of Compound A was prepared. The solution of Compound A was orally administered immediately before and 20 hours after intravitreal injection of thrombin at a dose of 1 mg/kg. In a vehicle administration group, a 1% (w/v) methyl cellulose solution was administered in the same manner.

(Evaluation Method)

At 24 hours after intravitreal injection of thrombin, each rat was killed by exsanguination, and then, the eyeball of the rat was enucleated while avoiding contamination with blood. After the eyeball was enucleated, a small cut was made in the vicinity of the optic papilla using a surgical knife, and the vitreous body was promptly collected. The collected vitreous body was appropriately diluted with purified water and the protein concentration was determined by the Bradford method. The thus determined protein concentration in the vitreous body was used as an index of retinal vascular permeability. Then, the suppression rate (%) of the drug to be evaluated against the retinal vascular hyperpermeability induced by thrombin was calculated in accordance with Equation 5. The result of Compound A is shown in Table 3. The case number in each administration group was 7 to 8 per group, and the mean value was used for calculating the suppression rate.

[Equation 5]

$$\text{Suppression rate against retinal vascular permeability (\%)} = (P_Y - P_Z) / (P_Y - P_X) \times 100$$

$P_x$: protein concentration in vitreous body in normal (untreated) group
$P_y$: protein concentration in vitreous body in group with intravitreal injection of thrombin and administration of vehicle
$P_z$: protein concentration in vitreous body in group with intravitreal injection of thrombin and administration of drug

Table 3

| Group | Suppression rate (%) |
|---|---|
| Compound A 1 mg/kg | 9.9 |

(Discussion)

From the above result, it was shown that Compound A has an excellent suppressive effect on retinal vascular hyperpermeability and has a marked prophylactic or improvement effect on a posterior ocular disease associated with retinal angiopathy such as diabetic retinopathy or diabetic macular edema.

[Preparation Examples]

The medicinal agent of the present invention will be more specifically described with reference to preparation examples, however, the present invention is not limited only to these preparation examples.

Formulation Example 1: Eye drop

| in 100 ml | |
|---|---|
| Compound A | 10 mg |
| Sodium chloride | 900 mg |
| Polysorbate 80 | q.s. |
| Disodium hydrogen phosphate | q.s. |
| Sodium dihydrogen phosphate | q.s. |
| Sterile purified water | q.s. |

Compound A and the other above-mentioned ingredients are added to sterile purified water, and these ingredients are mixed well, whereby an eye drop is prepared. By changing the addition amount of Compound A, an eye drop containing Compound A at a concentration of 0.05% (w/v), 0.1% (w/v), 0.5% (w/v) or 1% (w/v) can be prepared.

Formulation Example 2: Ophthalmic ointment

| in 100 g | |
|---|---|
| Compound A | 0.3 g |
| Liquid paraffin | 10.0 g |
| White petrolatum | q.s. |

Compound A is added to uniformly melted white petrolatum and liquid paraffin, these ingredients are mixed

well, and the resulting mixture is gradually cooled, whereby an ophthalmic ointment is prepared. By changing the addition amount of Compound A, an ophthalmic ointment containing Compound A at a concentration of 0.05% (w/w), 0.1% (w/w), 0.5% (w/w) or 1% (w/w) can be prepared.

Formulation Example 3: Tablet

| | |
|---|---|
| in 100 mg | |
| Compound A | 1 mg |
| Lactose | 66.4 mg |
| Cornstarch | 20 mg |
| Carxboxymethyl cellulose calcium | 6 mg |
| Hydroxypropyl cellulose | 6 mg |
| Magnesium stearate | 0.6 mg |

Compound A and lactose are mixed in a mixer, carboxymethyl cellulose calcium and hydroxypropyl cellulose are added thereto, and the resulting mixture is granulated. The obtained granules are dried, followed by sizing. Then, magnesium stearate is added and mixed with the sized granules and the resulting mixture is tableted with a tableting machine. By changing the addition amount of Compound A, a tablet containing Compound A in an amount of 0.1 mg, 10 mg or 50 mg in 100 mg of tablet can be prepared.

Formulation Example 4: Injection

| | |
|---|---|
| in 10 ml | |
| Compound A | 10 mg |
| Sodium chloride | 90 mg |
| Polysorbate 80 | q.s. |
| Sterile purified water | q.s. |

Compound A and sodium chloride are dissolved in sterile purified water, whereby an injection is prepared. By changing the addition amount of Compound A, an injection containing Compound A in an amount of 0.1 mg, 10 mg or 50 mg in 10 ml of injection can be prepared.

Industrial Applicability

Pramipexole or a salt thereof exhibits excellent inhibitory effect on neovascularization, suppressive effect on photoreceptor cell damage and suppressive effect on vascular hyperpermeability in a posterior ocular tissue such as choroid or retina, and is therefore useful as a prophylactic or therapeutic agent for a posterior ocular disease such as age-related macular degeneration, diabetic retinopathy or diabetic macular edema.

## Claims

1. A composition comprising a selective $D_2$ receptor agonist as an active ingredient, wherein the agonist is (S)-pramipexole or a salt thereof, for use in the prophylactic or therapeutic treatment of a posterior ocular disease.

2. The composition for use according to claim 1, wherein the posterior ocular disease is a disease in the vitreous body, retina, choroid, sclera or optic nerve.

3. The composition for use according to claim 1, wherein the posterior ocular disease is age-related macular degeneration, diabetic retinopathy, diabetic macular edema, retinitis pigmentosa, proliferative vitreoretinopathy, retinal artery occlusion, retinal vein occlusion, uveitis, Leber’s disease, retinopathy of prematurity, retinal detachment, retinal pigment epithelial detachment, central serous chorioretinopathy, central exudative chorioretinopathy, polypoidal choroidal vasculopathy, multifocal choroiditis, neovascular maculopathy, retinal artery macroaneurysm, optic nerve damage caused by any of these diseases or ischemic optic nerve damage.

4. The composition for use according to claim 1, wherein the posterior ocular disease is age-related macular degeneration, diabetic retinopathy or diabetic macular edema.

5. The composition for use according to claim 4, wherein the age-related macular degeneration is exudative age-related macular degeneration or atrophic age-related macular degeneration.

6. The composition for use according to any one of claims 1 to 5, wherein the route of administration is instillation administration, intravitreal administration, subconjunctival administration, administration into conjunctival sac, sub-Tenon's administration or oral administration.

7. The composition for use according to any one of claims 1 to 6, wherein the dosage form is an eye drop, an ophthalmic ointment, an insert, a patch, an injection, a tablet, a fine granule or a capsule.

8. Use of (S)-pramipexole or a salt thereof for manufacturing the prophylactic or therapeutic agent for a posterior ocular disease according to any one of claims 1 to 7.

**Patentansprüche**

1. Zusammensetzung, umfassend einen selektiven $D_2$-Rezeptor-Agonisten als aktiven Bestandteil, wobei der Agonist (S)-Pramipexol oder ein Salz davon ist, zur Verwendung in der prophylaktischen oder therapeutischen Behandlung einer posterioren Augenerkrankung.

2. Zusammensetzung zur Verwendung nach Anspruch 1, wobei die posteriore Augenerkrankung eine Erkrankung des Glaskörpers, der Retina, der Choroidea, der Sklera oder des Sehnervs ist.

3. Zusammensetzung zur Verwendung nach Anspruch 1, wobei die posteriore Augenerkrankung altersbedingte Makuladegeneration, diabetische Retinopathie, diabetisches Makulaödem, Retinitis pigmentosa, proliferative Vitreoretinopathie, Verschluß einer retinalen Arterie, Verschluß einer retinalen Vene, Uveitis, die Leber'sche Erkrankung, Retinopathia praematurorum, Netzhautablösung, Ablösung des retinalen Pigmentepithels, zentrale seröse Chorioretinopathie, zentrale exudative Chorioretinopathie, polypoidale choroidale Vaskulopathie, multifokale Choroiditis, neovaskuläre Makulopathie, Makroaneurysma einer retinalen Arterie, Schädigung des Sehnervs, verursacht durch eine dieser Erkrankungen oder ischämische Schädigung des Sehnervs ist.

4. Zusammensetzung zur Verwendung nach Anspruch 1, wobei die posteriore Augenerkrankung altersbedingte Makuladegeneration, diabetische Retinopathie oder diabetisches Makulaödem ist.

5. Zusammensetzung zur Verwendung nach Anspruch 4, wobei die altersbedingte Makuladegeneration exudative altersbedingte Makuladegeneration oder atrophische altersbedingte Makuladegeneration ist.

6. Zusammensetzung zur Verwendung nach einem der Ansprüche 1 bis 5, wobei der Verabreichungsweg Instillationsverabreichung, intravitreale Verabreichung, subkonjunktivale Verabreichung, Verabreichung in den Bindehautsack, sub-Tenon-Verabreichung oder orale Verabreichung ist.

7. Zusammensetzung zur Verwendung nach einem der Ansprüche 1 bis 6, wobei die Dosierungsform Augentropfen, eine Augensalbe, ein Einsatz, ein Pflaster, eine Injektion, eine Tablette, ein Feingranulat oder eine Kapsel ist.

8. Verwendung von (S)-Pramipexol oder eines Salzes davon zur Herstellung des prophylaktischen oder therapeutischen Mittels für eine posteriore Augenerkrankung nach einem der Ansprüche 1 bis 7.

**Revendications**

1. Composition comprenant un agoniste sélectif du récepteur $D_2$ comme ingrédient actif, où l'agoniste est le (S)-pramipexole ou un sel de celui-ci, à utiliser dans le traitement prophylactique ou thérapeutique d'une maladie oculaire

postérieure.

2. Composition à utiliser selon la revendication 1, où la maladie oculaire postérieure est une maladie du corps vitré, de la rétine, de la choroïde, de la sclère ou du nerf optique.

3. Composition à utiliser selon la revendication 1, où la maladie oculaire postérieure est la dégénérescence maculaire liée à l'âge, la rétinopathie diabétique, l'oedème maculaire diabétique, la rétinite pigmentaire, la vitréo-rétinopathie proliférative, l'occlusion de l'artère rétinienne, l'occlusion de la veine rétinienne, l'uvéite, la maladie de Leber, la rétinopathie de prématurité, le décollement de la rétine, le décollement de l'épithélium pigmentaire rétinien, la chorio-rétinopathie séreuse centrale, la chorio-rétinopathie exsudative centrale, la vasculopathie polypoïdale choroïdienne, la choroïdite multifocale, la maculopathie néovasculaire, le macroanévrisme rétinien artériel, un dommage du nerf optique provoqué par l'une quelconques de ces maladies ou un dommage ischémique du nerf optique.

4. Composition à utiliser selon la revendication 1, où la maladie oculaire postérieure est la dégénérescence maculaire liée à l'âge, la rétinopathie diabétique, l'oedème maculaire diabétique.

5. Composition à utiliser selon la revendication 4, où la dégénérescence maculaire liée à l'âge est une dégénérescence maculaire exsudative liée à l'âge ou une dégénérescence maculaire atrophique liée à l'âge.

6. Composition à utiliser selon l'une quelconque des revendications 1 à 5, où la voie d'administration est une administration par instillation, une administration intravitréenne, une administration sousconjonctivale, une administration dans le sac conjonctival, une administration de sous-ténonienne ou une administration orale.

7. Composition à utiliser selon l'une quelconque des revendications 1 à 5, où la présentation est une goutte oculaire, un onguent ophtalmique, un insert, un timbre, une injection, un comprimé, un granulé fin ou une capsule.

8. Utilisation du (S)-pramipexole ou d'un sel de celui-ci pour la préparation de l'agent prophylactique ou thérapeutique pour une maladie oculaire postérieure selon l'une quelconque des revendications 1 à 7.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 9101136 A
- JP 5072907 B


### Non-patent literature cited in the description


- *Nature Med.,* 2001, vol. 7, 569-574
- *Cancer Res.,* 2004, vol. 64, 5551-5555
- *Clinical Cancer Research,* 2004, vol. 10, 4349-4356
- *Journal of Japanese Ophthalmological Society,* 1999, vol. 103, 923-947
- Vitreoretinal Diseases. Shin Zusetsu Rinsho Ganka Koza. MEDICAL VIEW, 2000, 184-189232-237
- Ohan Shikkan Tekisuto & Atorasu. Igaku-Shoin Ltd, 43-45
- *Invest. Ophthalmol. Vis. Sci.,* 2005, vol. 46, 979-987
- *Journal of Japanese Ophthalmological Society,* 1989, vol. 93, 978-985